# EUROPEAN PATENT APPLICATION

(11) **EP 2 570 185 A1**
(43) Date of publication of application: **20.03.2013**
(21) Application number: 11181415.8
(22) Date of filing: 15.09.2011
(51) Int. Cl.: B01J 20/32, B01J 20/287, B01J 20/289

(54) **Sorbent comprising an aromatic ring system on its surface for the purification of organic molecules**

(71) Applicant: InstrAction GmbH, 68199 Mannheim (DE)
(72) Inventor: Arendt, Markus, Dr., 68766 Hockenheim (DE); Degel, Björn, Dr., 67454 Hassloch (DE); Schwarz, Thomas, Dr., 42799 Leichlingen (DE); Stumm, Gerhard, Dr., 22143 Hamburg (DE); Welter, Martin, Dr., 69118 Heidelberg (DE)
(74) Representative: Stolmár & Partner

(57) **Abstract**

The present invention relates to a sorbent comprising a solid support material, the surface of which comprises a residue of a general formula (I), wherein the residue is attached via a covalent single bond to a functional group on the surface of either the bulk solid support material itself or of a polymer film on the surface of the solid support material. Furthermore, the present invention relates to the use of the sorbent according to the invention for the purification of organic molecules, in particular pharmaceutically active compounds, preferably in chromatographic applications.

## Description

The present invention relates to a sorbent comprising a solid support material, the surface of which comprises a residue of a general formula (I), wherein the residue is attached via a covalent single bond to a functional group on the surface of either the bulk solid support material itself or of a polymer film on the surface of the solid support material. Furthermore, the present invention relates to the use of the sorbent according to the invention for the purification of organic molecules, in particular pharmaceutically active compounds, preferably in chromatographic applications.

Chromatography media for organic molecules and biomolecules have traditionally been categorized according to one or more of the following possible modes of interaction with a sample:
- hydrophobic interaction (reversed phase)
- hydrophilic interaction (normal phase)
- cation exchange
- anion exchange
- size exclusion
- metal ion chelation.

The provision of new chemical compounds, either by its discovery in plant extracts or animals or, by chemical synthesis, always demands the provision of new chromatographic materials, the further development of known chromatographic materials or the finding of a new way for the purification of the chemical compounds which is simple and cost-effective. That is, there is always a demand for new highly selective downstream purification technologies capable of handling large capacities without up-scaling the required volumes of liquid by the same factor.

Traditional stepwise application of the above chromatographic categories to a given separation problem was accordingly mirrored in a step-by-step, steady improvement of the product purity but also in product losses at every stage which accumulate seriously in the end, not to mention the operational time and cost of goods. Introduction of affinity chromatography at an early stage into the downstream process could be an answer to this demand since the reduction of a consecutive series of sequential chromatography steps into only one could thus be demonstrated many times. Affinity chromatography is sometimes regarded as a class of its own although, from a chemical point of view, it is based on the same interaction modes as above, but usually on a combination of two or more modes. By using affinity chromatography the specific interactions between an analyte and the sorbent may be verified both between the analyte and active residues bound on the surface of a matrix of the chromatographic material and between the analyte and surface characteristics of the matrix itself.

Affinity chromatography has mostly been carried out with bulk gel-phase resins. Pre-eminent gel-forming materials are medium-crosslinked polysaccharides, polyacrylamides, and poly(ethylene oxides). Such hydrogels often ensure a compatible interface which can well accommodate both the active residue of the ligand and the analyte interacting therewith due to their softness (conformational flexibility, elastic modulus), large pore systems, high polarity and high water content, as well as the absence of reactive or denaturing chemical groups. They are able to retain analytes, such as proteins, in their native state, i.e. preserve their correctly folded, three-dimensional structure, state of association, and functional integrity, or do not chemically change the structure of a complex pharmaceutically active compound. The mechanical resistance of these media is, however, much weaker than that of inorganic support materials since they are compressible under an applied pressure and do not tolerate shear stress caused by agitation, column packing or high liquid flow rates. Affinity sorbents that are fully compatible with robust HPLC process conditions are therefore rare.

Only in the recent past it has been recognised that the mechanical resistance of the stationary phase is a bulk property of the sorbent support whereas only a thin layer at the interface between the stationary and the mobile phases is responsible for mass exchange and for the interaction with the biological analyte. Therefore the concept of combining the function of a mechanically very rigid and dimensionally stable, porous 3-dimensional core, and a biocompatible, gellike interface layer which carries the active residues for binding the analyte has been brought up, and the associated synthetic problems have been technically solved. Such hybrid materials employ loosely crosslinked polymers of high polarity on a base of either an inorganic oxide or a densely crosslinked polymer of low polarity.

It was an object of the present invention to provide a new sorbent for chromatographic applications which allows the simple and cost-effective purification of organic molecules, even when used in chromatographic applications which demand a high stability of the material either with regard to the mechanic stress or in view of the solution characteristics of the eluent.

The present invention therefore provides a sorbent comprising a solid support material, the surface of which comprises a residue of the following general formula (I): 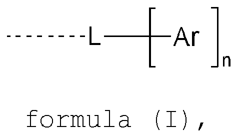 wherein the residue is attached via a covalent single bond represented by the dotted line in formula (I) to a functional group on the surface of either the bulk solid support material itself or of a polymer film on the surface of the solid support material; and
wherein the used symbols and indices have the following meanings:
- L: is an (n+1)-valent linear aliphatic hydrocarbon group having 1 to 30 carbon atoms or branched or cyclic aliphatic hydrocarbon group having 3 to 30 carbon atoms, wherein one or more CH₂-moieties in said groups may be substituted by a CO, NH, O or S, one or more CH-moieties in said groups may be substituted by N, said groups may comprise one or more double bonds between two carbon atoms, and one or more hydrogen atoms may be substituted by D, F, Cl or OH;
- Ar: represents independently at each occurrence a monovalent mono- or polycyclic aromatic ring system having 6 to 28 aromatic ring atoms, wherein one or more hydrogen atoms may be substituted by D, F, Cl, OH, C₁₋₆-alkyl, C₁₋₆-alkoxy, NH₂, -NO₂, -B(OH)₂, -CN or -NC; and
- n: is an index representing the number of Ar-moieties bound to L and is 1, 2 or 3.

An (n+1)-valent linear aliphatic hydrocarbon group having 1 to 30 carbon atoms or branched or cyclic aliphatic hydrocarbon group having 3 to 30 carbon atoms preferably is one of the following groups: methylene, ethylene, n-propylene, isopropylene, n-butylene, iso-butylene, sec-butylene (1-methylpropylene), tert-butylene, iso-pentylene, n-pentylene, tert-pentylene (1,1- dimethylpropylene), 1,2-dimethylpropylene, 2,2-dimethylpropylene (neopentylene), 1-ethylpropylene, 2-methylbutylene, n-hexylene, iso-hexylene, 1,2-dimethylbutylene, 1-ethyl-1-methylpropylene, 1-ethyl-2-methylpropylene, 1,1,2-trimethylpropylene, 1,2,2-trimethylpropylene, 1-ethylbutylene, 1-methylbutylene, 1,1-dimethylbutylene, 2,2-dimethylbutylene, 1,3-dimethylbutylene, 2,3-dimethylbutylene, 3,3-dimethylbutylene, 2-ethylbutylene, 1-methylpentylene, 2-methylpentylene, 3-methylpentylene, cyclopentylene, cyclohexylene, cycloheptylene, cyclooctylene, 2-ethylhexylene, trifluormethylene, pentafluorethylene, 2,2,2-trifluorethylene, ethenylene, propenylene, butenylene, pentenylene, cyclopentenylene, hexenylene, cyclohexenylene, heptenylene, cycloheptenylene, octenylene or cyclooctenylene, wherein
one or more CH₂-moieties in said groups may be substituted by a CO, NH, O or S,
one or more CH-moieties in said groups may be substituted by N,
said groups may comprise one or more double bonds between two carbon atoms, and
one or more hydrogen atoms may be substituted by D, F, Cl or OH.

It is preferred that L is an (n+1)-valent linear aliphatic hydrocarbon group having 1 to 20 carbon atoms, even more preferred 1 to 10 carbon atoms, or branched or cyclic aliphatic hydrocarbon group having 3 to 20 carbon atoms, even more preferred 3 to 10 carbon atoms,
wherein
one or more CH₂-moieties in said groups may be substituted by a CO, NH, O or S,
one or more CH-moieties in said groups may be substituted by N,
said groups may comprise one or more double bonds between two carbon atoms, and
one or more hydrogen atoms may be substituted by D, F, Cl or OH;

L is even more preferably an n-valent linking unit selected from the group consisting of
- (C₁₋₁₀-alkylene)-,
- (C₁₋₆-alkylene)-NH-,
- C(O)-,
- C(O)-NH-,
- C(O)-CH(OH)-,
- C(O)-NH-NH-C(O)O-,
- C(O)-(C₁₋₁₂-alkylene) -,
- C(O)-NH-(C₁₋₆-alkylene)-,
- C(O)-(C₁-₁₂-alkylene)-C(O)-,
- C(O)-(C₁₋₁₂-alkylene)-NH-C(O)O-,
- C(O)-(C₁₋₆-alkylene)-C(O)-NH-,
- C(O)-(C₁₋₆-alkylene)-C(O)-NH-(C₁₋₆-alkylene)-,
- C(O)-O-(C₁₋₆-alkylene)-,
- C(O)-(C₁₋₆-alkylene)-Y-, wherein Y is NH, O or S,
- C(O)-(C₁₋₃-alkylene)-O-(C₁₋₃-alkylene)-C(O)-NH-,
- C(O)-(C₁₋₃-alkylene)-O-(C₁₋₃-alkylene)-C(O)-NH-(C₁-₆-alkylene)-,
- C(O)-(C₁₋₆-alkylene)-C(O)-NH-(C₁₋₆-alkylene)-NH-C(O)-NH-,
- CH₂-CH(OH)-CH₂-(OCH₂CH₂)ₘ-O-, wherein m is 1, 2, 3, 4, 5 or 6;
- (C₁₋₆-alkylene)-Y-(C₁-₆-alkylene)-, wherein Y is S, O, NH or -S(O₂)-;
- C(O)-(CH(CH₂CH(CH₃)₂))-NH-C(O)-,
- C(O)-NH-(C₁₋₆-alkylene)-NH-C(O)-,
- C(O)-(C₁₋₆-alkylene)-NH-C(O)-(CH(CH₂CH(CH₃)₂))-NH-C(O)-, 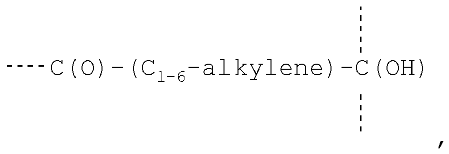 and

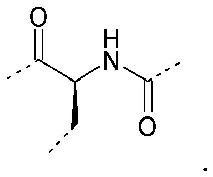

It is further preferred that L is
- C(O)-,
- C(O)CH₂-,
- C(O)CH₂CH₂-,
- C(O)CH₂CH₂CH₂-,
- C(O)-CH=CH-,
- C(O)CH(OH)-,
- C(O)CH(CH₃)-,
- C(O)CH₂O-,
- C(O)NH-,
- C(O)NHCH₂-,
- C(O)NHCH(CH₃),
- CH₂CH₂-,
- (CH₂)₄-NH-,
- C(O)CH₂CH₂C(O)-,
- C(O)CH₂CH₂C(O)-NH-,
- C(O)CH₂CH₂C(O)NHCH₂-,
- C(O)CH₂CH₂C(O)NHCH₂CH₂-,
- C(O)CH₂CH₂C(O)NHCH₂CH₂CH₂-,
- C(O)CH₂CH₂C(O)NHCH₂CH₂NHC(O)NH-,
- C(O)OCH₂-,
- C(O)OCH₂CH₂-,
- C(O)CH₂S-,
- C(O)CH₂OCH₂C(O)NHCH₂-,
- CH₂CH₂S (O)₂CH₂CH₂-,
- CH₂CH(OH) CH₂OCH₂CH₂OCH₂CH (OH) CH₂-,
- CH₂CH(OH)CH₂(OCH₂CH₂)₅O-,
- C(O)(CH₂)₁₀-,
- C(O)(CH(CH₂CH(CH₃)₂))-NH-C(O)-,
- C(O)(CH₂CH₂CH₂)-NH-C(O)-(CH(CH₂CH(CH₃)₂))-NH-C(O)-, 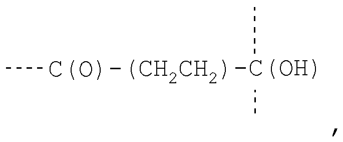 or

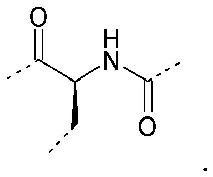

It is further more preferred that L is
- C(O)-,
- CH₂CH₂-,
- C(O)NH-,
- C(O)NHCH₂-,
- C(O)CH₂O-,
- C(O)CH₂CH₂-,
- C(O)CH₂CH₂CH₂-,
- C(O)CH₂CH₂C(O)NH-,
- (CH₂)₄-NH-,
- C(O)CH₂CH₂C(O)NH-CH₂-,
- C(O)CH₂CH₂C(O)NH-CH₂CH₂,
- C(O)CH₂CH₂C(O)NHCH₂CH₂NHC(O)NH-,
- C(O)OCH₂-,
- C(O)CH₂OCH₂C(O)NHCH₂-,
- CH₂CH (OH) CH₂ (OCH₂CH₂) ₅O-,
- C(O)-(CH(CH₂CH(CH₃)₂))-NH-C(O)-,
- C(O)CH(OH)-,
- C(O)CH(CH₃)-,
- C(O)NHCH(CH₃)-,
- C(O)-(CH₂CH₂CH₂)-NH-C(O)-(CH(CH₂CH(CH₃)₂))-NH-C(O)-, 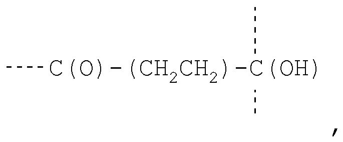 or 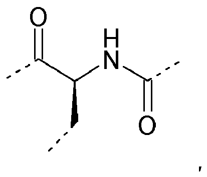
wherein the dotted lines in all above mentioned definitions of L represent the bonds to the functional group of the solid support material or of the polymer film and Ar, and wherein in all above listed linkers L it is preferred that the first mentioned atom having a free ending line is connected in this position to the solid support material.

It is even more preferred that L is -C(O)-, -CH₂CH₂-, - C(O)CH₂O- or -C(O)NH-, wherein the units are connected to the functional group via its carbonyl atom; -C(O) and -C(O)NH-being most preferred.

A monovalent mono- or polycyclic aromatic ring system in the sense of the present invention is preferably an aromatic ring system having 6 to 28 carbon atoms as aromatic ring atoms. Under the term "aromatic ring system" a system is to be understood which does not necessarily contain only aromatic groups, but also systems wherein more than one aromatic units may be connected or interrupted by short non-aromatic units (< 10 % of the atoms different from H, preferably < 5 % of the atoms different from H), such as sp³-hybridized C, O, N, etc. or -C(O)-. These aromatic ring systems may be mono- or polycyclic, i.e. they may comprise one (e.g. phenyl) or two (e.g. naphthyl) or more (e.g. biphenyl) aromatic rings, which may be condensed or not, or may be a combination of condensed and covalently connected rings. The aromatic atoms of the ring systems may be substituted with D, F, Cl, OH, C₁₋₆-alkyl, C₁₋₆-alkoxy, NH₂, -NO₂, -B(OH)₂, -CN or -NC.

Preferred aromatic ring systems e.g. are: phenyl, biphenyl, triphenyl, naphthyl, anthracyl, binaphthyl, phenanthryl, dihydrophenanthryl, pyrene, dihydropyrene, chrysene, perylene, tetracene, pentacene, benzpyrene, fluorine, indene and ferrocenyl.

It is further preferred that Ar is a monovalent aromatic ring system having 6 to 14 aromatic ring atoms, which may be substituted or not. That is, it is more preferred that Ar is phenyl, naphthyl, anthracyl or pyryl, which may be substituted or not. It is even more preferred that either no hydrogen atom of Ar is substituted or one or more hydrogen atoms of Ar is/are substituted by one or more of F or CN. It is even more preferred that Ar is a perfluorated aromatic ring system, preferably a perfluorated phenyl. Alternatively, Ar may be substituted with one -CN. In this case Ar may be a phenyl which is substituted with -CN, preferably in para-position with respect to the position of L.

The residues according to formula (I) may in a preferred way be all combinations of preferred and most preferred meanings for L and the most preferred meanings of Ar.

Furthermore, it is preferred that n is 1 or 2, even more preferred 1, so that L is a bivalent linker.

Preferred examples of the residues of formula (I) are the following: 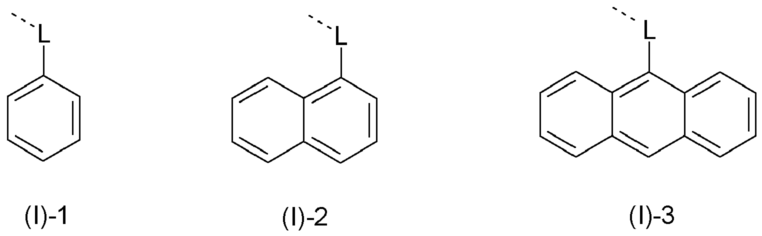 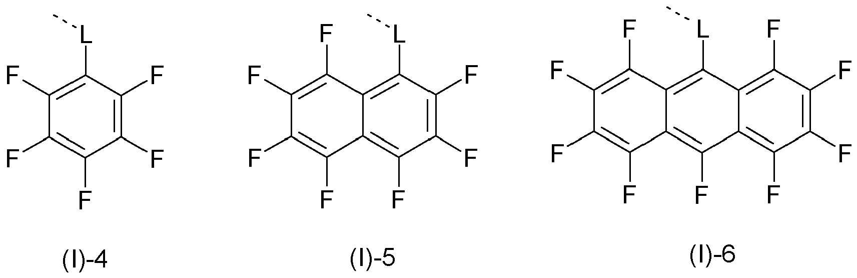 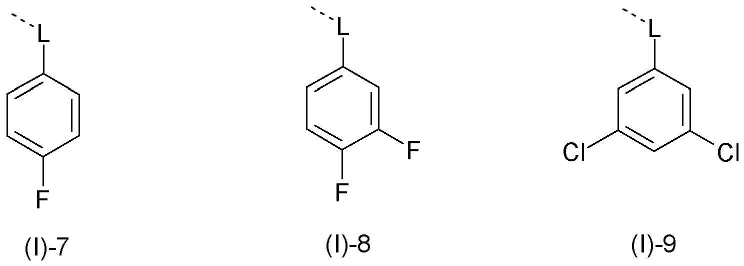 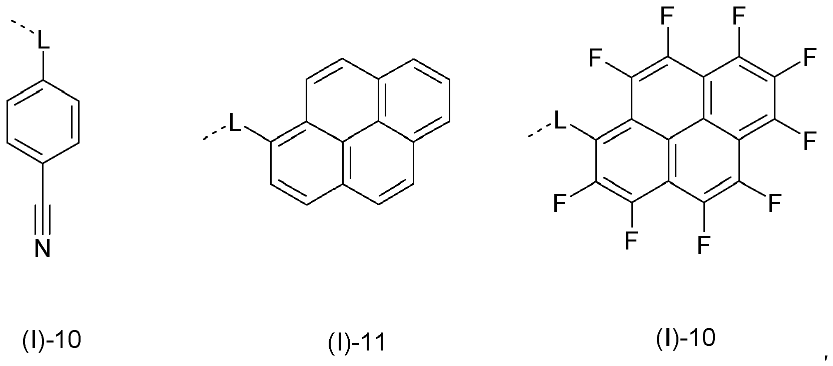 wherein L has the same general and preferred meanings as defined above, and wherein (I)-4, (I)-5, (I)-6, (I) -7, (I)-8, (I)-9 and (I)-10 are even more preferred, and wherein (I)-4 and (I)-10 are the most preferred.

The most preferred residues of formula (I) are the following:

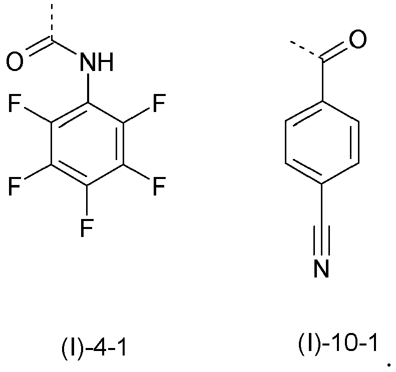

The sorbent according to the invention may further comprise a further residue comprising a group P_{S}, which is either a deprotonizable or an anionic group, wherein the further residue is also attached via a covalent single bond to a functional group on the surface of either the bulk solid support material itself or of the polymer film on the surface of the solid support material, depending on whether the solid support may comprise a polymer film or not.

It is particularly preferred that, if the residues of formula (I) are bound to the functional group which is on the surface of the solid support material itself, the sorbent according to the invention comprises the further residue comprising a group P_{S}.

The group Pₛ is either an anionic group or a group which may become an anionic group in solution. It is preferred that these groups are totally or partly present as ionic groups in a ph range of between 6 and 8. The groups Pₛ may also be polar groups having a hydrogen atom which can be split off by means of stronger bases, wherein these hydrogen atoms are preferably bound to a heteroatom.

Examples of the groups Pₛ are as follows:
a) -COOH, -SO₃H, -CONH₂, -CONHNH₂, -SO₂NH₂, -PO₃H₂, - PO (OH) (NH₂), -CO(NHOH), -CO (NH (O-C₁₋₄-Alkyl)), -CSNH₂, - NHCONH₂, -N(OH)CONH₂, -NHCSNH₂, -CSNHNH₂;
b) 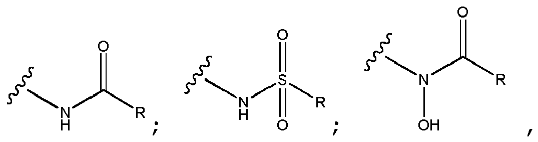 wherein R = -(C₁₋₄-alkyl), -O(C₁₋₄alkyl), -NH(C₁₋₄alkyl), (substituted) aryl, (substituted) O-aryl, (substituted) NH-aryl, -CF₃ and other fluorated alkyl groups;
c) 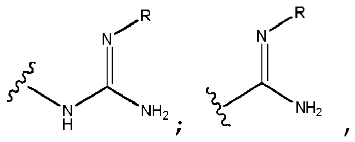 wherein R = -OH, -CN, -NO₂;
d) 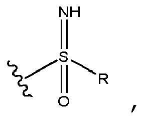 wherein R = (C₁₋₄-alkyl), (substituted) aryl, - CF₃ and other fluorated alkyl groups;
e) 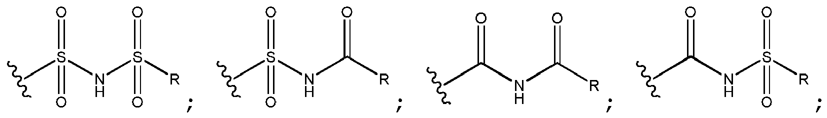 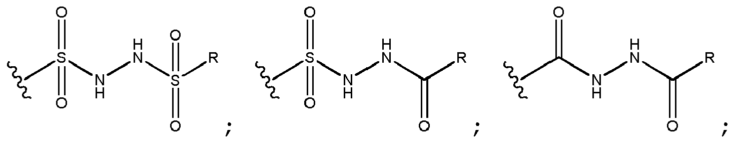 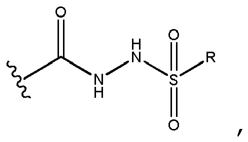 wherein R = -(C₁₋₄-alkyl), -O(C₁₋₄-alkyl), -NH(C₁₋₄alkyl), -NH (C₂₋₄-alkenyl), (substituted) aryl, (substituted) O-aryl, (substituted) NH-aryl, -CF₃ and other fluorated alkyl groups;
f) 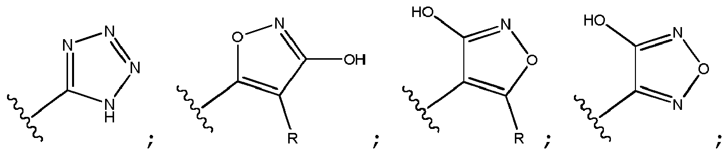 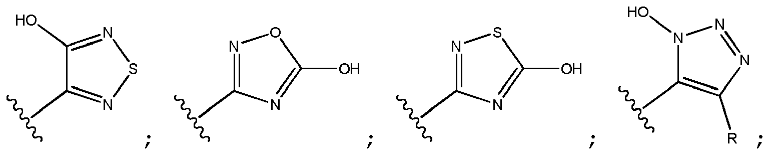 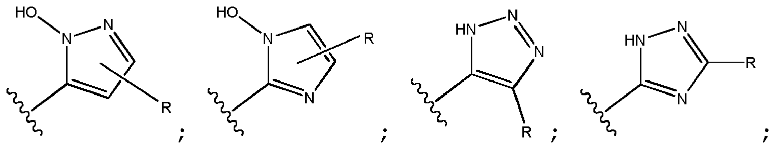 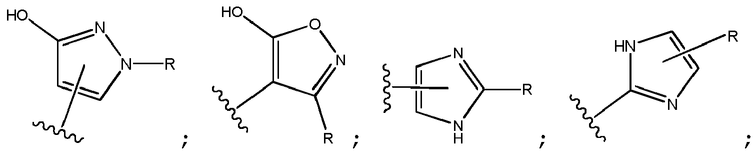 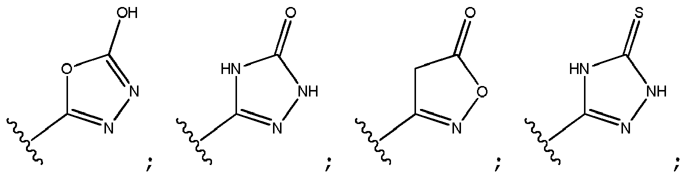 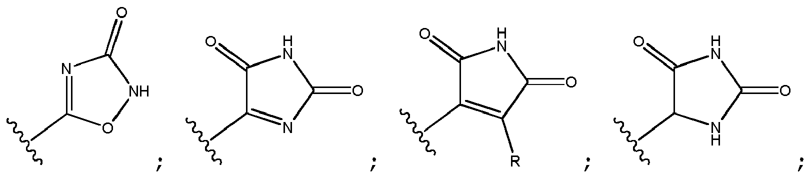 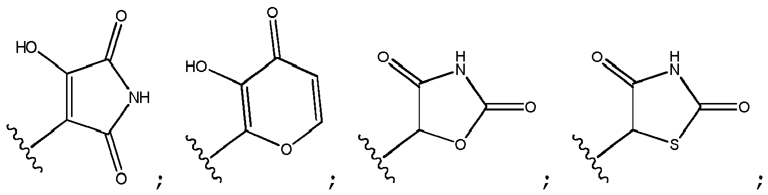 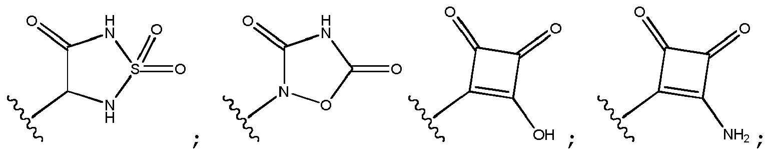 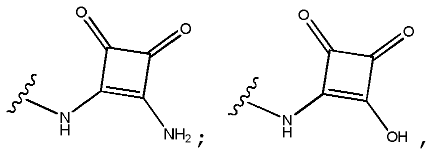 wherein R = H, -(C₁₋₄-alkyl), -CF₃ and other fluorated alkyl groups;
g) -OH and -SH.

It is more preferred that the group P_{S} is -SO₃H, -COOH or -PO₃H₂, and even more preferred -SO₃H or -COOH.

The residue comprising a group Pₛ is preferably a residue of the following formulae (IIa) or (IIb): 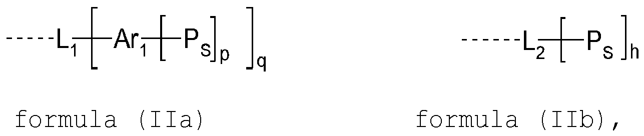 wherein the residue is attached via a covalent single bond represented by the dotted line in formula (IIa) or (IIb) to a functional group on the surface of either the bulk solid support material itself or of a polymer film on the surface of the solid support material, depending on whether the solid support material comprises a polymeric film or not; and
wherein the used symbols and parameters have the following meanings:
- L₁: is a (q+1)-valent linker which has the same general meanings as defined above for L;
- L₂: is a (h+1)-valent linker which has the same general meaning as defined above for L;
- Ar₁: represents independently at each occurrence a (p+1)-valent mono- or polycyclic aromatic ring system having 6 to 28, preferably 6 to 14, most preferred 6, aromatic ring atoms or a (p+1)-valent mono- or polycyclic heteroaromatic ring system having 5 to 28, preferably 5 to 14, most preferred 5 aromatic ring atoms, wherein one or more hydrogen atoms of the aromatic or heteroaromatic ring system may be substituted by a residue R₁;
- R₁: is selected from the group consisting of C₁₋₆-alkyl, C₁₋₆-alkoxy, D, F, Cl, Br, -CN, -NC and -C₁₋₆-alkyl esters of carboxylic, phosphoric or boronic acids;
- p: is 1, 2 or 3, more preferred 1 or 2 and most preferred 1;
- h: is 1, 2 or 3, more preferred 1 or 2 and most preferred 1;
- q: is 1 or 2, more preferred 1.

L₁ is preferably selected from the group consisting of
- C(O)-,
- C(O)CH₂-,
- C(O)CH₂CH₂-,
- C(O)CH₂CH₂CH₂-,
- C(O)-CH=CH-,
- C(O)CH(OH)-,
- C(O)CH(CH₃)-,
- C(O)CH₂O-,
- C(O)NH-,
- C(O)NHCH₂-,
- C(O)NHCH(CH₃)-,
- CH₂CH₂-,
- (CH₂)₄-NH-,
- C (O) CH₂CH₂C (O)-,
- C(O)CH₂CH₂C(O)-NH-,
- C (O) CH₂CH₂C (O) NHCH₂-,
- C (O) CH₂CH₂C (O) NHCH₂CH₂-,
- C (O) CH₂CH₂C (O) NHCH₂CH₂CH₂-,
- C (O) CH₂CH₂C (O) NHCH₂CH₂NHC (O) NH-,
- C (O) OCH₂-,
- C (O) OCH₂CH₂-,
- C (O) CH₂S-,
- C (O) CH₂OCH₂C (O) NHCH₂-,
- CH₂CH₂S (O) ₂CH₂CH₂-,
- CH₂CH (OH) CH₂OCH₂CH₂OCH₂CH (OH) CH₂-,
- CH₂CH (OH) CH₂ (OCH₂CH₂) ₅O-,
- C (O) (CH₂) ₁₀-,
- C (O) - (CH (CH₂CH (CH₃)₂))-NH-C (O)-,
- C (O) - (CH₂CH₂CH₂) -NH-C (O) - (CH (CH₂CH(CH₃)₂))-NH-C (O)-,
- C(O) -CH (CH₂CH₂CH₂NHC (=NH) NH₂) NHC (O)-, 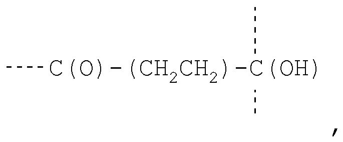 or

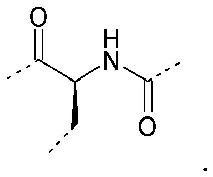

It is further more preferred that L₁ is
- C(O)-,
- CH₂CH₂-,
- C (O) NH-,
- C (O) NHCH₂-,
- C (O) CH₂O-,
- C (O) CH₂CH₂-,
- C (O) CH₂CH₂CH₂-,
- C (O) CH₂CH₂C (O) NH-,
- (CH₂) ₄-NH-,
- C (O) CH₂CH₂C (O) NH-CH₂-,
- C (O) CH₂CH₂C (O) NH-CH₂CH₂,
- C (O) CH₂CH₂C (O) NHCH₂CH₂NHC (O) NH-,
- C (O) OCH₂-,
- C (O) CH₂OCH₂C (O) NHCH₂-,
- CH₂CH (OH) CH₂ (OCH₂CH₂) ₅O-,
- C (O) - (CH (CH₂CH(CH₃) ₂)) -NH-C (O)-,
- C (O) CH (OH)-,
- C (O) CH (CH₃)-,
- C (O) NHCH (CH₃)-,
- C (O) - (CH₂CH₂CH₂) -NH-C (O) - (CH(CH₂CH(CH₃)₂)) -NH-C(O)-,
- C (O) -CH (CH₂CH₂CH₂NHC (=NH)NH₂)NHC (O)- , 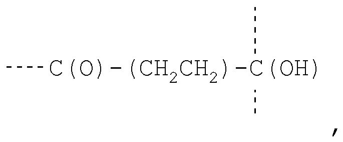 or 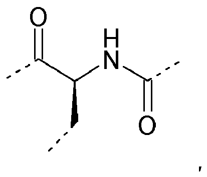
wherein in all definitions above of L₁ the dotted lines represent the bonds to the functional group of the solid support material or the polymer film and Ar₁, and wherein in all above listed linkers L₁ it is preferred that the first mentioned atom having a free ending line is connected in this position to the solid support material.

It is even more preferred that L₁ is -C(O)-, -CH₂CH₂-, -C (O) CH₂O- or -C(O)NH-, wherein the units are connected to the functional group via its carbonyl atom, -C(O)- being most preferred.

L₂ is preferably selected from the group consisting of
- (C₁₋₆-alkylene) -,
- C (O) - (C₁₋₆-alkylene) -C(O)NH- (C₁₋₆-alkylene)-,
- C (O) - (C₁-₆-alkylene) -,
- C (O) - CH(NH (C (O) OC (CH₃)₃)) - (C₁₋₃-alkylene) -,
- C (O) CH (NH₂) (C₁₋₃-alkylene) -,
- C (O) -CH(NH (C(=NH) (NH₂)))-(C₁₋₆-alkylene)-,
- C (0) - (C₁₋₃-alkylene) -C(=CH₂)-,
- C (O) C(=CH₂) - (C₁₋₃-alkylene)-,
- C(O)CH=CH-,
- C (O) - (C₁₋₃-alkylene) -CH (OH)-(C₁₋₃-alkylene)-,
- C (O) - (C₁₋₃-alkylene) CH=CH-,
- C (O) - (C₁₋₃-alkylene) CH (CH₂OH)-,
- C (O) - (C₁₋₃-alkylene) -C (=CH₂)-, 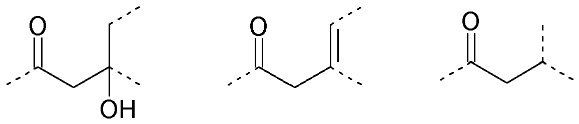 and

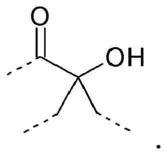

L₂ is more preferably selected from the group consisting of
- CH₂CH₂CH₂-,
- C (O) CH₂-,
- C (O) CH₂CH₂-,
- C (O) CH₂CH₂CH₂-,
- C (O) CH₂CH₂C (O) NHCH₂CH₂-,
- C (O) -CH (NH₂) CH₂-,
- C (O) -CH (NH(C(O)OC(CH₃)₃)) CH₂-,
- C (O) CH₂OCH₂-,
- C (O) CH₂C (=CH₂) -,
- C (O) C (=CH₂) CH₂-,
- C (O) CH=CH-,
- C (O) CH₂CH (OH) CH₂-,
- C (O) CH₂CH=CH-,
- C (O) CH₂CH (CH₂OH)-,
- C (O) CH₂C (=CH₂)-, 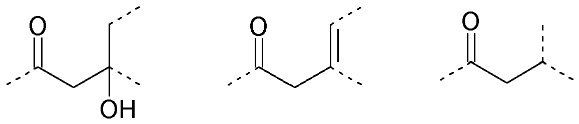 and 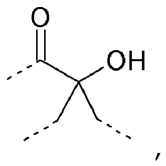
wherein the dotted lines in all above listed linkers L₂ represent the bonds to the functional group of the solid support material or the polymer film and Pₛ, and wherein in all above listed linkers L₂ it is preferred that the first mentioned atom having a free ending line is connected in this position to the solid support material.

L₂ is even more preferred -C (O) - (C₁₋₆-alkylene)-, and most preferred -C (O) CH₂CH₂-.

In case of a residue according to formula (IIb) Pₛ is preferably -SO₃H or -COOH, more preferred -COOH.

The most preferred residue according to formula (IIb) is the following:

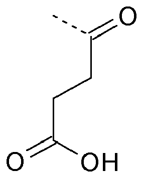

A (p+1)-valent mono- or polycyclic aromatic ring system having 6 to 28, preferably 6 to 14, most preferred 6, aromatic ring atoms in the sense of the present invention is preferably an aromatic ring system having 6 to 28, preferably 6 to 14, most preferred 6 carbon atoms as aromatic ring atoms. Under the term "aromatic ring system" a system is to be understood which does not necessarily contain only aromatic groups, but also systems wherein more than one aromatic unit may be connected or interrupted by short non-aromatic units (< 10 % of the atoms different from H, preferably < 5 % of the atoms different from H), such as sp³-hybridized C, O, N, etc. or - C(O)-. These aromatic ring systems may be mono- or polycyclic, i.e. they may comprise one (e.g. phenyl) or two (e.g. naphthyl) or more (e.g. biphenyl) aromatic rings, which may be condensed or not, or may be a combination of condensed and covalently connected rings.

Preferred aromatic ring systems e.g. are: phenyl, biphenyl, triphenyl, naphthyl, anthracyl, binaphthyl, phenanthryl, dihydrophenanthryl, pyrene, dihydropyrene, chrysene, perylene, tetracene, pentacene, benzpyrene, fluorine, indene and ferrocenyl.

It is further preferred that Ar₁ is phenyl, naphthyl, anthracyl, pyryl or perylyl, phenyl and naphthyl being even more preferred. In accordance with the definition of the index p, Ar₁ may be substituted with one, two or three groups Pₛ which may be the same or may be different. It is preferred that, when p is 2 or 3, the groups Pₛ may either be the same or may be a combination of -COOH and -SO₃H.

A (p+1)-valent mono- or polycyclic heteroaromatic ring system having 5 to 28, preferably 5 to 14, most preferred 5 aromatic ring atoms in the sense of the present invention is preferably an aromatic ring system having 5 to 28, preferably 5 to 14, most preferred 5 atoms as aromatic ring atoms. The heteroaromatic ring system contains at least one heteroatom selected from N, O, S and Se (remaining atoms are carbon). Under the term "heteroaromatic ring system" a system is to be understood which does not necessarily contain only aromatic and/or heteroaromatic groups, but also systems wherein more than one (hetero)aromatic unit may be connected or interrupted by short non-aromatic units (< 10 % of the atoms different from H, preferably < 5 % of the atoms different from H), such as sp³-hybridized C, O, N, etc. or -C(O)-. These heteroaromatic ring systems may be mono- or polycyclic, i.e. they may comprise one (e.g. pyridyl) or two or more aromatic rings, which may be condensed or not, or may be a combination of condensed and covalently connected rings.

Preferred heteroaromatic ring systems are for instance 5-membered rings, such as pyrrole, pyrazole, imidazole, 1,2,3-triazole, 1,2,4-triazole, tetrazole, furane,thiophene, selenophene, oxazole, isoxazole, 1,2-thiazole, 1,3-thiazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 6-membered rings, such as pyridine, pyridazine, pyrimidine, pyrazine, 1,3,5-triazine, 1,2,4-triazine, 1,2,3-triazin, 1,2,4,5-tetrazine, 1,2,3,4-tetrazine, 1,2,3,5-tetrazine, or condensed groups, such as indole, isoindole, indolizine, indazole, benzimidazole, benzotriazole, purine, naphthimidazole, phenanthrimidazole, pyridimidazole, pyrazinimidazole, chinoxalinimidazole, benzoxazole, naphthoxazole, anthroxazole, phenanthroxazole, isoxazole, benzothiazole, benzofurane, isobenzofurane, dibenzofurane, chinoline, isochinoline, pteridine, benzo-5,6-chinoline, benzo-6,7-chinoline, benzo-7,8-chinoline, benzoisochinoline, acridine, phenothiazine, phenoxazine, benzopyridazine, benzopyrimidine, chinoxaline, phenazine, naphthyridine, azacarbazole, benzocarboline, phenanthridine, phenanthroline, thieno[2,3b]thiophene, thieno[3,2b]thiophene, dithienothiophene, isobenzothiophene, dibenzothiophene, benzothiadiazothiophene or combinations of these groups. Even more preferred are imidazole, benzimidazole and pyridine.

It is, however, preferred that Ar₁ is a (p+1)-valent mono- or polycyclic aromatic rings system.

Examples for residues according to formula (IIa) are as follows: 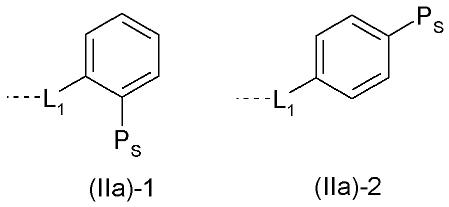 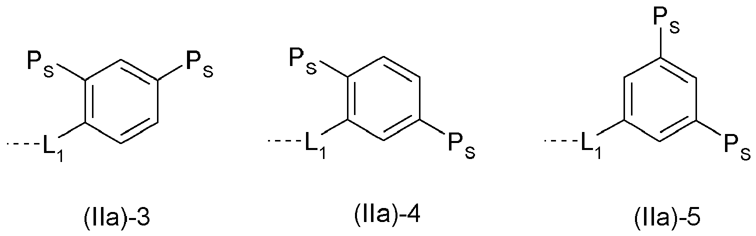 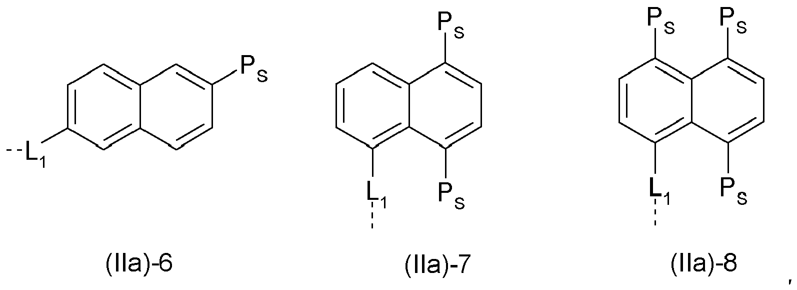 wherein the residue with formula (IIa)-1 is particularly preferred.

In case of a residue according to formula (IIa) Pₛ is preferably -COOH or -SO₃H, more preferred -SO₃H.

The most preferred residue according to formula (IIa) is the following:

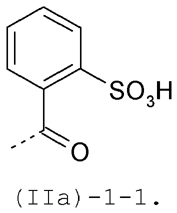

In an embodiment the sorbent of the present invention does not comprise any further residue than the residue according to formula (I).

In an embodiment the sorbent of the present invention comprises residues according to formula (I) and residues according to formula (IIa). In this embodiment it is further preferred that Ar in formula (I) is a perfluorated aromatic ring system, perfluorated phenyl as Ar being more preferred. Furthermore, in this embodiment it is further preferred that Ar₁ is phenyl. Independently, but preferred in combination of the preferred variants of this embodiment, P_{S} is -SO₃H.

In an embodiment the sorbent of the present invention comprises one residue according to formula (I) of the following structure 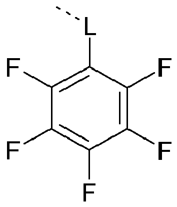 and one residue according to formula (IIa) of the following structure 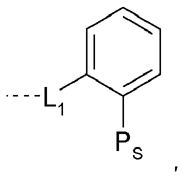 wherein L, L₁ and P_{S} independently of each other preferably - but not limited to - have the following meanings:
- L: is -C (O) -NH-, wherein N binds to Ar,
- L₁: is -C (O) -, and
- Pₛ: is -SO₃H.

It is further preferred in the before-mentioned embodiment that all of the symbols L, L₁ and Pₛ have meanings as defined in this embodiment.

In case the sorbent according to the invention comprises residues according to formula (IIa) and residues according to formula (I), the ratio per mole of a residue according to formula (IIa) to a residue according to formula (I) is preferably in the range of from 0.01 to 1, more preferably from 0,03 to 0,5, still more preferred from 0,05 to 0,3, still more preferred 0,08 to 0,2 and most preferred 0,1.

In an embodiment the sorbent of the present invention comprises residues according to formula (I) and residues according to formula (IIa). In this embodiment it is further preferred that Ar in formula (I) is a perfluorated aromatic ring system, phenyl as Ar being more preferred. Furthermore, in this embodiment it is further preferred that Ar₁ is phenyl. Indpendently, but preferred in combination of the preferred variants of this embodiment, Pₛ is -SO₃H.

In an embodiment the sorbent of the present invention comprises one residue according to formula (I) of the following structure 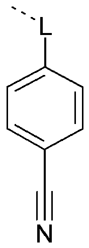 and
one residue according to formula (IIb) of the following structure

------ L₂―Pₛ

wherein L, L₂ and Pₛ independently of each other - but not limited to - have the following meanings:
- L: is -C (O),
- L₂: is -C (O) - (C₁₋₆-alkylene)-, wherein -C (O) CH₂CH₂- is most preferred,
- Pₛ: is -COOH.

It is further preferred in the before-mentioned embodiment that all of the symbols L, L₂ and Ps have (preferred) meanings as defined.

In case the sorbent according to the invention comprises residues according to formula (IIb) and residues according to formula (I), the ratio per mole of a residue according to formula (IIb) to a residue according to formula (I) is preferably in the range of from 0.01 to 2, more preferably from 0,05 to 1,5, still more preferred from 0,1 to 1, still more preferred 0,3 to 0,8 and most preferred 0,5.

According to the present invention a C₁₋₆-alkyl is a linear, branched or cyclic alkyl group. Linear alkyl groups have preferably 1 bis 6, more preferably 1 to 3 carbon atoms. Branched or cyclic alkyl groups preferably have 3 to 6 carbon atoms. One or more hydrogen atoms of these alkyl groups may be substituted with fluorine atoms. Furthermore, one or more CH₂-groups may be substituted with NR, O or S (R is preferably H or C₁₋₆-alkyl). If one or more CH₂ groups are substituted with NR, O or S, it is preferred that only one of these groups are substituted; even more preferred substituted by an O-atom. Examples of these compounds comprise the following: methyl, ethyl, n-propyl, i-propyl, n-butyl, i-butyl, s-butyl, t-butyl, 2-methylbutyl, n-pentyl, s-pentyl, cyclopentyl, n-hexyl, cyclohexyl, n-heptyl, cycloheptyl, n-octyl, cyclooctyl, 2-ethylhexyl, trifluormethyl, pentafluorethyl and 2,2,2-trifluorethyl.

A C₁₋₆-alkoxy is a C₁₋₆-alkyl group which is connected via an o-atom.

A C₁₋₁₂-alkylene, C₁₋₁₀-alkylene, C₁₋₆-alkylene or C₁₋₃-alkylene is an alkyl groups as defined above, wherein one hydrogen atom is not present and the resulting bivalent unit has two bonds.

A C₂₋₄-alkenyl is a linear or branched alkenyl group with 2 to 4 carbon atoms. One or more hydrogen atoms of these alkenyl groups may be substituted with fluorine atoms. Furthermore, one or more CH₂-groups may be substituted by NR, O or S (R is preferably H or C₁₋₆-alkyl). If one or more CH₂-groups are substituted by NR, O or S, it is preferred that only one of these groups are substituted; even more preferred substituted by an O-atom. Examples of these groups are ethenyl, propenyl and butenyl.

An aryl is a mono- or polycyclic aromatic or heteroaromatic hydrocarbon residue which preferably contains 5 to 20, more preferred 5 to 10 and most preferred 5 or 6 aromatic ring atoms. If this unit is an aromatic unit it contains preferably 6 to 20, more preferred 6 to 10 and most preferred 6 carbon atoms as ring atoms. If this unit is a heteroaromatic unit it contains preferably 5 to 20, more preferred 5 to 10 and most preferred 5 carbon atoms as ring atoms. The heteroatoms are preferably selected from N, O and/or S. A (hetero)aromatic unit is either a simple aromatic cycle, such as benzene, or a simple heteroaromatic cycle, such as pyridine, pyrimidine, thiophene, etc., or a condensed aryl- or heteroaryl group, such as naphthaline, anthracene, phenanthrene, chinoline, isochinoline, benzothiophene, benzofurane and indole, and so on.

Examples for (hetero)aromatic units are as follows: benzene, naphthalene, anthracene, phenanthrene, pyrene, chrysene, benzanthracene, perylene, naphthacene, pentacene, benzpyrene, furane, benzofurane, isobenzofurane, dibenzofurane, thiophene, benzothiophene, isobenzothiophene, dibenzothiophene, pyrrole, indole, isoindole, pyridine, chinoline, isochinoline, acridine, phenanthridine, benzo-5,6-chinoline, benzo-6,7-chinoline, benzo-7,8-chinoline, phenothiazine, phenoxazine, pyrazole, indazole, imidazole, benzimidazole, naphthimidazole, phenanthrimidazole, pyridimidazole, pyrazinimidazole, chinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzpyrimidine, chinoxaline, 1,5-diazaanthracene, 2,7-diazapyrene, 2,3-siazapyrene, 1,6-diazapyrene, 1,8-diazapyrene, 4,5-diazapyrene, 4,5,9,10-tetraazaperylene, pyrazine, phenazin, phenoxazine, phenothiazine, fluorubine, naphthyridine, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

The solid support material is preferably a macroporous material. The pore size of the solid support material is preferably at least 50 nm, more preferably from 50 to 400 nm and most preferably from 150 to 250 nm.

According to an embodiment of the sorbent according to the invention, the solid support material has a specific surface area of from 1 m²/g to 1000 m²/g, more preferred of from 30 m²/g to 800 m²/g and most preferred of from 50 to 500 m²/g.

It is preferred that the solid support material has a porosity of from 30 to 80 % by volume, more preferred from 40 to 70 % by volume and most preferred from 50 to 60 % by volume. The porosity can be determined by mercury intrusion according to DIN 66133. The pore size of the solid support material can also be determined by pore filling with the mercury intrusion method according to DIN 66133. The specific surface area can be determined by nitrogen adsorption with the BET-method according to DIN 66132.

The solid support material may be an organic polymeric material or an inorganic material. Especially in case that the sorbent according to the invention comprises more than one residue, the solid support material is preferably an inorganic material.

In case the solid support material is a polymeric material, it is substantially non-swellable. For that reason, it is mostly preferred that the polymeric material has a high crosslinking degree.

The polymeric material is preferably crosslinked at a degree of at least 5 %, more preferably at least 10 % and most preferably at least 15 %, based on the total number of crosslinkable groups in the polymeric material. Preferably, the crosslinking degree of the polymeric material does not exceed 50 %.

Preferably the polymeric material for the solid support material is selected from the group consisting of generic or surface-modified polystyrene, (e.g. poly(styrene-co- dinvinylbenzene)), polystyrene sulfonic acid, polyacrylates, polymethacrylates, polyacrylamides, polyvinylalcohol, polysaccharides (such as starch, cellulose, cellulose esters, amylose, agarose, sepharose, mannan, xanthan and dextran), and mixtures thereof.

The polymeric material possibly used in the present invention preferably has before the crosslinking has been performed 10 to 10000, particularly preferably 20 to 5000 and very particularly preferably 50 to 2000 repeat units. The molecular weight M_{W} of the polymeric material before the crosslinking has been performed is preferably in the range of 10000 to 2000000 g/mol, particularly preferably in the range of 100000 to 1500000 g/mol, and very particularly preferably in the range of 200000 to 1000000 g/mol. The determination of M_{W} can be performed according to standard techniques known to the person skilled in the art by employing gel permeation chromatography (GPC) with polystyrene as internal standard, for instance.

In case the solid support material is an inorganic material, the inorganic material is some kind of inorganic mineral oxide, preferably selected from the group consisting of silica, alumina, magnesia, titania, zirconia, fluorosile, magnetite, zeolites, silicates (cellite, kieselguhr), mica, hydroxyapatite, fluoroapatite, metal-organic frameworks, ceramics and glasses, like controlled pore glass (e.g. trisoperl), metals such as aluminium, silicon, iron, titanium, copper, silver, gold and also graphite or amorphous carbon.

Independent of whether the solid support material is a polymeric material or an inorganic material, the solid support material provides a solid base of a minimum rigidity and hardness which functions as an insoluble support and provides a basis for the enlargement of the interface between stationary and mobile phases which is the place of interaction with the analyte as the molecular basis for the process of the partitioning between said phases, and for an increased mechanical strength and abrasiveness, especially under flow and/or pressurized conditions.

The solid support materials according to the invention may be of homogeneous or heterogeneous composition, and therefore also incorporate materials which are compositions of one or more of the materials mentioned above, in particular multilayered composites.

The solid support material may be a particulate material, preferably having a particle size of from 5 to 500 µm. The solid support material may also be a sheet- or fibre-like material such as a membrane. The external surface of the solid support material thus may be flat (plates, sheets, foils, disks, slides, filters, membranes, woven or nonwoven fabrics, paper) or curved (either concave or convex: spheres, beads, grains, (hollow) fibres, tubes, capillaries, vials, wells in a sample tray).

The pore structure of the internal surface of the solid support material may, inter alia, consist of regular, continuous capillary channels or of cavities of irregular (fractal) geometry. Microscopically, it can be smooth or rough, depending on the way of manufacture. The pore system can either extend continuously throughout the entire solid support material or end in (branched) cavities. The rate of an analyte's interfacial equilibration between its solvation in the mobile phase and its retention on the surface of the stationary phase and thus the efficiency of a continuous flow separation system is largely determined by mass transfer via diffusion through the pores of the solid support material and thus by its characteristic distribution of particle and pore sizes. Pore sizes may optionally show up as asymmetric, multimodal and / or spatially (e.g. cross-sectionally) inhomogeneous distributions.

In one embodiment, the surface of the solid support material may not be covered with a further material, such as a polymer. In this case the residues of formulae (I), and optionally (IIa) and (IIb), bind to a surface group (functional group) of the solid support material itself. In this case the following solid support materials are preferred: silicagel with alkylsilanol groups containing functional groups, such as a hydroxy group or an amine group, for attaching ligands (i.e. residues according to formula (I) or (II)), aromatic polymers like styrene polymers with functionalized aromatic groups containing amines or carboxylic acids, polymethylmetacrylates with partially cleaved ester groups for attaching ligands.

In one embodiment, the inorganic support is preferred when the residues directly bind to functional groups which are part of the surface of the solid support material itself.

Alternatively, the surface of the solid support material may preferably be covered with a film of a polymer which comprises or consists of individual chains which are preferably covalently crosslinked with each other, but which are preferably not covalently bound to the surface of the solid support material.

As described above, the macroporous support has a crosslinked polymer on the surface of the macroporous support. The crosslinked polymer may be covalently bound with the macroporous support or be adhered to the macroporous support. Preferably, the crosslinked polymer is adhered to the macroporous support.

The preferred polymer for the crosslinkable polymer comprises at least one polymer containing amino groups. Polyvinylamine is strongly preferred. Other suitable polyamines may comprise polyethylene imine, polyallylamine etc. as well as functional polymers other than those containing amino groups, such as polyvinyl alcohol, polyvinyl acetate, polyacrylic acid, polymethacrylic acid, their precursor polymers such as poly(maleic anhydride), polyamides, or polysaccharides (cellulose, dextran, pullulan etc.).

If co-polymers are employed, the preferred co-monomers are simple alkene monomers or polar, inert monomers like vinyl pyrrolidone.

The polymer used for the film on the solid support material is most preferably a polyamine, a polyvinylamine, or a copolymer or a polymer blend comprising polyamine.

The polymer can be applied to the macroporous support by all means of coating known to a person skilled in the art such as absorption, vapor phase deposition, polymerization from the liquid, gas or plasma phase, spin coating, surface condensation, wetting, soaking, dipping, rushing, spraying, damping, evaporation, application of electric fields or pressure, as well as methods based on molecular self-assembly such as, for example, liquid crystals, Langmuir Blodgett- or layer-by-layer film formation. The polymer may thereby be coated directly as a monolayer or as multilayer or as a stepwise sequence of individual monolayers on top of each other.

According to a preferred embodiment of the sorbent according to the invention, the crosslinking degree of the crosslinked polymer is at least 2 %, based on the total number of crosslinkable groups in the crosslinked polymer. More preferred the crosslinking degree is of from 5 to 50 %, more preferred of from 5 to 30 %, most preferred from 10 to 20 %, based on the total number of crosslinkable groups in the crosslinked polymer. The crosslinking degree can easily be adjusted by the stoichiometric amount of the crosslinking reagent used. It is assumed that nearly 100 mol% of the crosslinker reacts and forms crosslinks. This can be verified by analytical methods. The crosslinking degree can be determined by MAS-NMR spectroscopy and quantitative determination of the amount of crosslinker in relation to the amount of polymer. This method is most preferred. The crosslinking degree can also be determined by IR spectroscopy based on e.g. C-O-C or OH vibrations using a calibration curve. Both methods are standard analytical methods for a person skilled in the art.

The crosslinking reagent used for crosslinking the polymer is preferably selected from the group consisting of dicarboxylic acids, diamines, diols and bis-epoxides. In one embodiment the at least one crosslinking reagent is a linear, conformationally flexible molecule of a length of between 1 and 20 atoms.

Preferred molecular weights of the polymers used range from, but are not limited to, 5000 to 50000 g/mol, which is particularly true for polyvinylamine. Polymers having a molecular weight near the lower limit of the range given above have shown to penetrate even narrow pores of the carrier so that solid state materials with high surface areas and consequently with good mass transfer kinetics, resolution and binding capacity can be used in the sorbents of the present invention.

According to a further embodiment the crosslinked polymer carries functional groups.

The term "functional group" means any simple, distinct chemical moiety belonging to the crosslinked polymer on the surface of the solid support material or to the crosslinkable polymer during preparation of a polymer film on the surface of the solid support material. Thereby, the functional group may serve as chemical attachment point or anchor. Functional groups preferably contain at least one weak bond and/or one heteroatom, preferably a group behaving as nucleophil or electrophil.

The preferred functional groups are primary and secondary amino, hydroxyl, and carboxylic acid or ester groups, when taken before the residues of formulae (I), (IIa) or (IIb) have been bound to these groups. When the residues are bound to the functional groups the nature of these groups change with respect to the structure of the residues bound.

The invention also relates to a method for preparing a sorbent, preferably the sorbent according to the invention, comprising:
(i) providing a polymer having functional groups;
(ii) adsorbing a film of said polymer onto the surface of a carrier;
(iii) crosslinking a defined portion of said functional groups of the adsorbed polymer with at least one crosslinking reagent;
(iv) derivatising further defined portions of said functional groups of the crosslinked polymer with one or more residues according to the formulae (I), (IIa) or/and (IIb).

The polymer to be adsorbed on the surface of the carrier is preferably solved in an aqueous media wherein the pH is suitably adjusted in order to solve or suspend the polymer.

The adsorbing of the polymer on the surface of the carrier is preferably done by dipping the carrier into the solution or suspension containing the polymer. The mixture is then preferably shaked in order to get a complete mix of the ingredients. Capillaric forces make sure that pores of the carrier are soaked with the solution or suspension. Then, the water is preferably evaporated in vacuum at a temperature between 40 and 60°C, thereby depositing the polymer at the walls of the pores in the form of a film. Then, the coated material is preferably suspended in an organic solvent, such as isopropanol or dimethylformamide (DMF), and is preferably crosslinked by means of a crosslinking agent, such as ethylene glycol diglycidyl ether, preferably at a temperature between 25 and 60°C for 4 to 8 hours..

In the case, wherein the solid support material does not contain a polymeric material on its surface, the residues according to formulae (I), (IIa) and (IIb) bind directly to functional groups on the surface of the solid support material.

Depending on the kind of functional groups and depending on the residue according to formulae (I), (IIa) and (IIb) different derivatization strategies of the solid support can be used. If the solid support material contains amino groups as functional groups, residues containing a carboxylic acid group can be attached to the amine nitrogen atom via the carboxylic carbon atom via peptide chemistry using coupling reagents like 2- (1H-benzotriazole-1-yl) -1,1,3,3-tetramethyluronium hexafluorophosphate (HBTU), O- (1H-6-chlorobenzotriazole-1-yl)-1,1,3,3-tetramethyluronium hexafluorophosphate (HCTU), benzotriazole-1-yl-oxy-tris-pyrrolidino-phosphonium hexafluorophosphate (PyBOP), propylphosphonic anhydride (T3P) etc. or by using reactive groups in the reagent like isocyanates, epoxides or anhydrides. If the solid support material contains amino groups, aliphatic carbon atoms of the residue according to formulae (I), (IIa) and (IIb) may be bound to the amine nitrogen atom via a nucleophilic aliphatic substitution. In case the residue according to formula (IIa) or (IIb) contains carboxylic acid groups as group Pₛ, these groups have to be protected in order to ensure that the carboxylic acid group of the linker (before being attached to the solid support material) and not the group Pₛ binds to the functional group on the surface of the solid support material.

If the solid support material contains hydroxy groups, residues according to formulae (I), (IIa) and (IIb) containing a carboxylic acid group before being attached to the functional group may be attached to the oxygen atom of the hydroxy group via the carboxylic carbon atom by using the carboxylic acid chloride or the ester of the carboxylic acid group. If the solid support material contains hydroxy groups, aliphatic carbon atoms of the residue according to formulae (I), (IIa) and (IIb) may be bound to the oxygen atom of the hydroxy group via a nucleophilic aliphatic substitution.

If the solid support material contains carboxylic acid groups, carboxylic acid esters or carboxylic acid anhydrides, the residue according to formulae (I), (IIa) and (IIb) may be attached via nucleophilic attack of a nucleophilic group, such as -NH₂, -OH, -SH at the electrophilic carbon atom of the carboxylic acid group, acid ester or anhydride, thereby forming an amide, ester or thioester.

The sorbent of the present invention may be used for the purification of organic molecules (organic compounds) or the purification of solutions from certain organic molecules. That is, the present invention further refers to the use of a sorbent according to the invention for the purification of organic molecules or the purification of solutions from organic molecules.

The term "purification" is referred to as comprising separating, or increasing the concentration and/or purity of a organic molecule from a mixture containing said organic molecule.

In other words the present invention is also directed to a method of purification of organic molecules which also includes the separation of unwanted organic molecules from a solution by using the sorbent of the present invention.

The use of the sorbent according to the invention for the purification of organic molecules or separating organic molecules (organic compounds) or the method for the purification of organic molecules or separating organic molecules from a solution by using the sorbent according to the invention comprises the following steps:
(i) applying a crude mixture comprising the organic molecules being dissolved or suspended in a liquid on a chromatographic column containing the sorbent according to the invention or a sorbent prepared according to a method of the invention;
(ii) elution of the organic molecule from the column by using an eluent.

The eluent used in step (ii) may be the same solvent as used for the liquid in step (i), but may also be different, depending on the conditions necessary for the purification of the organic molecules. As liquid in step (i) or eluent in step (ii) every kind of solvent or buffering system applicable in the field of chromatography may be used. In the present invention the solvent may be pure water, mixtures of water with a water-soluble organic solvent, such as acetonitrile or alcohols having a low molecular weight, such as methanol or ethanol, or aqueous buffering systems often in combination with alcohols having a low molecular weight, such as methanol, ethanol. Organic acid salts and organic acids may be used as buffer, such as sodium formate or a combination of sodium formate with ascorbic acid.

The organic molecules purified by means of the sorbent of the present invention are preferably a pharmaceutically active compounds.

The organic nmolecules have preferably a molecular weight in the range of from 500 to 200000 g/mol, more preferably in the range of from 500 to 150000 g/mol, and most preferred of from 500 to 2500 g/mol.

Particularly preferred as organic molecules used in the use/process of the present invention are partricine, tacrolimus, irinotecane , voglibose and the derivatives thereof; the most preferably organic molecules have the following structures: 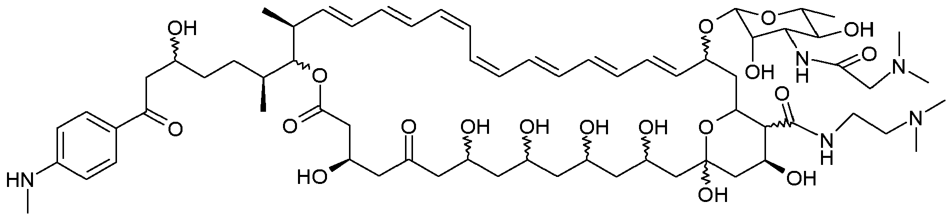 partricine derivative, 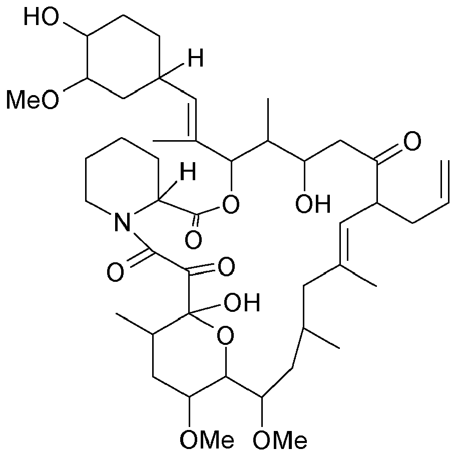 tacrolimus, 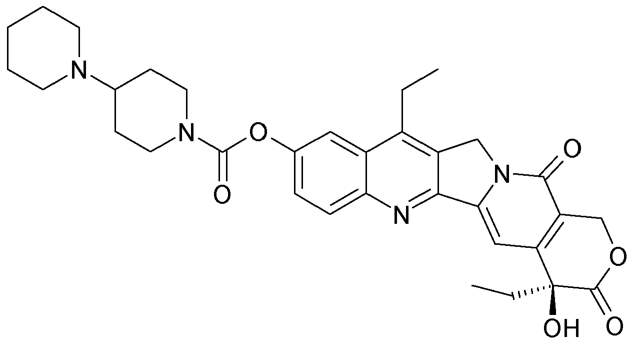 irinotecane, and 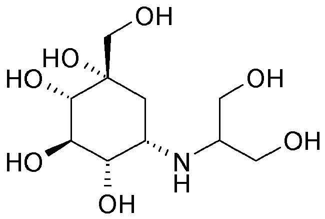 voglibose.

Furthermore, the sorbent according to the invention may also be used for separating endotoxines from solutions. The term "endotoxines" as used in the present invention refers to a class of biochemical substances. Endotoxines are decomposition products of bacteria, which may initiate variable physiologic reactions in humans. Endotoxines are components of the outer cell membrane (OM) of gram-negative bacteria or blue-green algae. From the chemical view endotoxines are lipopolysaccharides (LPS) which are composed of a hydrophilic polysaccharide component and a lipophilic lipide component. In contrast to the bacteria endotoxines stem from, endotoxines are very thermally stable and endure sterilisation. The currently most sensitive method of measuring endotoxines is made by means of the activation of the coagulation cascade in the lysate of amoebocytes which have been isolated from limulus polyphemus. This test is commonly known as the socalled LAL-test.

In the case of the purification of partricine, tacrolimus irinotecane or the derivatives thereof, preferably the partricine derivative, the tacrolimus or the irinotecane as shown above, it is preferred that a sorbent according to the invention is used which comprises a residue according to formula (I). In this case it is particularly preferred that the sorbent comprises a residue according to formula (I)-4-1.

In the case of the purification of tacrolimus, irinotecane or the derivatives thereof, preferably the tacrolimus or the irinotecane as shown above, it is preferred that a sorbent according to the invention is used which comprises only residues according to formula (I). In this case it is particularly preferred that the residue is that of formula (I)-4-1.

In the case of the purification of partricine or its derivatives, preferably the partricine derivative as shown above, it is preferred that a sorbent according to the invention is used which comprises a residue according to formula (I) and a residue according to formula (IIa). It is further preferred that the residue according to formula (I) is that of formula (I)-4-1 and that the residue according to formula (IIa) is that of formula (IIa)-1-1.

In the case of the purification of voglibose or its derivatives, preferably the voglibose as shown above, it is preferred that a sorbent according to the invention is used which comprises a residue according to formula (I). In this case it is particularly preferred that the sorbent comprises a residue according to formula (I)-10-1.

In the case of the purification of voglibose or its derivatives, preferably the voglibose as shown above, it is further preferred that a sorbent according to the invention is used which comprises a residue according to formula (I) and a residue according to formula (IIb). In this case it is particularly preferred that the residue according to formula (I) is that of formula (I)-10-1 and that the residue according formula (IIb) is -C (O) -CH₂CH₂COOH.

The invention also relates to a column for liquid chromatography or solid phase extraction comprising a sorbent according to the invention or a sorbent prepared according to a method according to the invention as a stationary phase within a tubular containment and optionally further components such as frits, filter plates, flow distributors, seals, fittings, screwings, valves, or other fluid handling or connection elements. In one embodiment, the method is further characterised by its physical and chemical resistance against applied pressures up to 20 bar, against applied heat up to 110 °C, as well as against common sanitisation protocols, thus enabling its repetitive use of up to 1,000 times, preferably up to 5,000 times. The invention also relates to a collection of a plurality of the same or different sorbents according to the invention or of sorbents prepared according to a method according to the invention or of columns according to the invention in the format of a microplate or microchip array, or a multi-capillary or microfluidic device, capable of being processed in parallel.

The invention also relates to a diagnostic or laboratory purification kit comprising a sorbent according to the invention or a sorbent prepared according to a method according to the invention or a column according to the invention or a collection of sorbents or columns according to the invention and, within the same packaging unit, further chemical or biological reagents and / or disposables necessary for carrying out the method according to the invention or a different analytical, diagnostic, or laboratory method different therefrom.

The present invention further refers to the following embodiments:
(i) A method for the purification of organic molecules by using a sorbent according to the invention.
(ii) The method according to embodiment (i), wherein the organic molecules are pharmaceutically active compounds.
(iii)The method according to embodiment (i) or (ii), wherein the organic molecules have a molecular weight in the range of from 500 to 200000 g/mol.
(iv) The method according to any one of embodiments (i) to (iii), wherein the organic molecules are selected from the group consisting of a partricine, tacrolimus, irinotecane, voglibose and endotoxines.

The present invention is further explained by means of the following figures and examples which should however not be understood as being limiting for the scope of the present invention:

### Figures:

- Fig. 1:: Chromatogram of a sample fractionation (Example 5) of a crude mixture of tacrolimus and several impurities separated by a sorbent according to the invention produced in Example 1.
- Fig. 2:: Analytical chromatogram overlay of the crude mixture (1) and the combined fractions R13-R16 (2) in Example (5).
- Fig. 3:: Chromatogram of a sample fractionation (Example 7) of a crude mixture of a derivative of partricine separated by a sorbent according to the invention produced in Example 2.
- Fig. 4:: Analytical chromatogram comparing the crude mixture (short dashed line) and the purified product (continuous line) together with a working standard (dashed and dotted line) in Example 7.
- Fig. 5:: Fractionation chromatogram of the purification of voglibose in Example 8.
- Fig. 6:: LC-MS analytics of the fractionated product (6a) and a mixture with the impurities (6b) in Example 8.
- Fig. 7:: Fractionation chromatogram of the purification of irinotecane in Example 6.

### Examples:

### Example 1: Method A of producing a sorbent according to the invention comprising a residue of formula (I)-4-1:

Silicagel SP-1000-10 from DAISO was coated with polyvinylamine using 66.7 g of a 12% polyvinylamine solution in water with adjusted pH between 9.0 to 9.5 for 100 g of silicagel. The mixture was agitated on a sieve shaker until the solution was fully soaked up in the pores of the silicagel. After that the sorbent was dried in vacuum at 50°C until the water was completely evaporated. Afterwards the dried sorbent was suspended in 150 mL N,N-Dimethylmethanamide (DMF) and agitated at 25°C for 16 hours with 1.28 g of 1,12-Bis-(5-norbornen-2,3-dicarboximido)-decandicarboxylic acid. Afterwards the sorbent was filtered off and washed with 230 mL DMF, 390 mL 0.5 M trifluoroacetic acid (TFA) in DMF, 780 mL 0.1 M TFA in H₂O, 230 mL H₂O and 230 mL MeOH. After drying the sorbent is ready for further modification.

3 g of the coated and crosslinked sorbent was suspended in 10 mL DMF, washed three times with 10 mL 0.5 M triethylamine (TEA) and suspended in 10 mL DMF again. 0.22 g pentafluorophenylene isocyanate was added and the mixture was agitated at 25° C for 4 hours. After that the mixture was filtered off and the sorbent was washed with 60 mL 0.5 M TEA in DMF, 40 mL DMF, 60 mL 0.5 M TEA in DMF and 40 mL DMF again. The sorbent was suspended in 10 mL DMF and 0.11 g pentafluorophenylene isocyanate was added. The mixture was again agitated for 18 hours at 25°C. After that the solution was filtered off and the sorbent washed with 50 mL DMF, 100 mL 0.5 M trifuoroacetic acid in water, 100 mL water and 100 mL methanol. After drying at 50°C in vacuum the sorbent is ready to use.

### Example 2: Method of producing a sorbent according to the invention comprising residues of formulae (I)-4-1 and (IIa)-1-1:

Silicagel Jupiter 300-15 from Phenomenex was coated with polyvinylamine using 20.4 g of a 10% polyvinylamine solution in water with adjusted pH between 9.0 to 9.5 for 25 g of silicagel. The mixture was agitated on a sieve shaker until the solution was fully soaked up in the pores of the silicagel. After that the sorbent was dried in vacuum at 50°C until the water was completely evaporated. Afterwards the dried sorbent was suspended in 150 mL N,N-Dimethylmethanamide (DMF) and agitated at 25°C for 16 hours with 1.28 g of 1,12-Bis-(5-norbornen-2,3-dicarboximido)-decandicarboxylic acid.

Afterwards the sorbent was filtered off and washed with 230 mL DMF, 390 mL 0.5 M TFA in DMF, 780 mL 0.1 M TFA in H₂O, 230 mL H₂O and 230 mL MeOH. After drying the sorbent is ready for further modification.

11 g of the coated and crosslinked sorbent was suspended in 30 mL DMF, washed three times with 30 mL 0.5 M triethylene amine and suspended in 30 mL DMF again. 2.4 g pentafluorophenylene isocyanate was added and the mixture was agitated at 25° C for 4 hours. After that the mixture was filtered off and the sorbent was washed with 60 mL 0.5 M TEA in DMF, 40 mL DMF, 60 mL 0.5 M TEA in DMF and 40 mL DMF again. The sorbent was suspended in 25 mL DMF and 0.76 g pentafluorophenylene isocyanate was added. The mixture was again agitated for 18 hours at 25°C. After that the solution was filtered off and the sorbent washed with 50 mL DMF, 100 mL 0.5 M trifluoroacetic acid in DMF, 50 mL DMF, 100 mL 0.5 M triethylene amine and 50 mL DMF.

For the introduction of the second ligand 3 g of the sorbent were suspended in 20 mL DMF. 0.57 g 2-sulfobenzoic acid anhydride and 0.43 mL triethylene amine were added and the mixture was agitated for 16 hours at 25°C. Afterwards the solution was filtered off and the sorbent washed with 140 mL DMF, 140 mL 0.5 M TFA in DMF, 140 mL DMF, 140 mL 0.5 M TEA in DMF and 140 mL DMF. The sorbent was suspended in 15 mL DMF again and agitated with 0.29 g 2-sulfobenzoic acid anhydride and 0.22 mL triethylene amine for 16 hours at 25°C. Afterwards the solution was filtered off and the sorbent washed with 140 mL 0.5 M TFA in DMF, 140 mL 0.5 M TFA in water, 140 mL water and 140 mL methanol. After that the sorbent was dried in vacuum at 50°C.

### Example 3: Method B of producing a sorbent according to the invention comprising a residue of formula (I)-4-1:

Silicagel Jupiter 300-10 was coated with polyvinylamine using 26.2 g of a 10% polyvinylamine solution in water diluted with additional 14.3 mL water and with adjusted pH at 9.25 for 32 g of silicagel. The mixture was agitated on a sieve shaker until the solution was fully soaked up in the pores of the silicagel. This was achieved in about 2.5 hours. After that the sorbent was dried in vacuum at 40°C until the water was completely evaporated. Afterwards the dried sorbent was suspended in 150 mL N,N-Dimethylmethanamide (DMF) and agitated at 25°C for 20 hours with 1.66 g of 1,12-Bis-(5-norbornen-2,3-dicarboximido)-decandicarboxylic acid. Afterwards the sorbent was filtered off and washed with 300 mL DMF, 500 mL 0.5 M TFA in DMF, 1000 mL 0.1 M TFA in H₂O, 300 mL H₂O and 300 mL MeOH. After drying the sorbent is ready for further modification. 10 g of the coated and crosslinked sorbent was suspended in 25 mL DMF, washed three times with 25 mL 0.5 M triethylenamine and suspended in 25 mL DMF again. 1.84 g pentafluorophenylene isocyanate was added and the mixture was agitated at 25° C for 4 hours. After that the mixture was filtered off and the sorbent was washed with 50 mL DMF, 100 mL 0.5 M TFA in DMF, 100 mL DMF, 100 mL 0.5 M TEA in DMF and 40 mL DMF again. The sorbent was suspended in 25 mL DMF and 0.81 g pentafluorophenylene isocyanate was added. The mixture was again agitated for 4 hours at 25°C. After that the solution was filtered off and the sorbent washed with 50 mL DMF, 100 mL 0.5 M trifluoroacetic acid in water, 100 mL water and 100 mL methanol. After drying at 40°C in vacuum the sorbent is ready for use.

### Example 4: Method of producing a sorbent according to the invention comprising residues of formula (I)-10-1 and residues of the following formula: -C (O) -CH₂CH₂COOH:

The coating and crosslinking of the sorbent was performed according to Example 1. Further modification was done as follows: 10 g of the sorbent was washed with 150 mL 0.5 M TEA in DMF and suspended afterwards in 30 mL DMF. 640 mg 4-cyanobenzoic acid, 1.72 g HBTU, 590 mg N-hydroxybenzotriazole (HOBt) and 605 µL TEA were diluted in 15 mL DMF and given to the suspension. The mixture was agitated for 12 hours and subsequently filtered off. The sorbent was washed with 150 mL DMF, 150 mL 0.1 M TFA in DMF, 150 mL DMF, 150 mL 0.5 M TEA in DMF and 150 mL DMF. Afterwards the sorbent was resuspended in 20 mL DMF and 213 mg 4-cyanobenzoic acid, 549 mg HBTU, 196 mg HOBt and 203 µL TEA were added. The mixture was agitated for 24 hours and subsequently washed with 100 mL DMF, 150 mL 0.5 M TFA in DMF, 150 mL DMF, 150 mL 0.5 M TEA in DMF and 150 mL DMF. Afterwards it was suspended in 30 mL DMF. 724 mg succinic acid anhydride and 1 mL TEA were added and the mixture agitated for 16 hours at 25°C. The washing and reaction step was performed three additional times before the sorbent was washed with 150 mL DMF, 150 mL 0,5 M TFA in DMF, 150 mL 0,5 M TFA in water, 150 mL water, 150 mL methanol and dried in vacuum at 50°C.

### Example 5: Purification of tacrolimus by using the sorbent produced in Example 1:

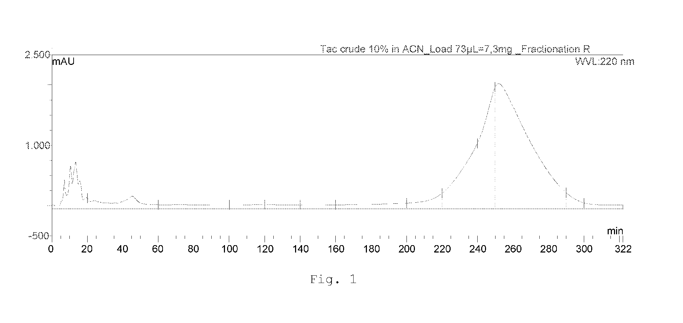

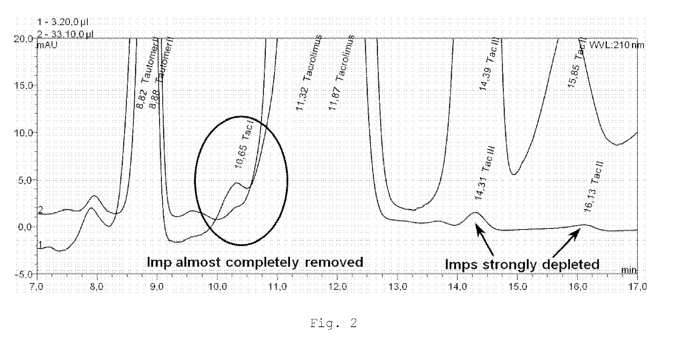

The crude mixture of tacrolimus and several impurities were separated using an Dionex HPLC system consisting of a four channel low-pressure gradient pump (LPG 580, LPG 680 or LPG 3400), auto sampler (Gina 50, ASI-100 or WPS-300), six-channel column switching valves (Besta), column oven and a diode-array uv detector (UVD 170U, UVD 340S or VWD 3400). The sorbent produced in Example 1 was filled in a 250x4 mm steel column. The method for separation was an isocratic fractionation with water/acetonitrile 70/30. The course of the fractionation is shown in Figure 1 and the analytices of the several fractions in the Table 1 below. Tacrolimus could be obtained by combining the fractions R13 to R16 in 90.4% purity and 85% yield with the major impurities the tautomer with 4.4% and the other impurities way below 1% (Table 1). Figure 2 shows the reduction of the main impurities from chromatogram 1 (crude) to chromatogram 2 (purified).

**Table 1: Analytical results of the fractionation:**

| | **Tacrolimus** | | **Tautomer II** | **Tac I** | **Tac II** | **Tac III** |
|---|---|---|---|---|---|---|
| **fraction** | **Purity [%]** | **Yield [%]** | **Purity [%]** | **Purity [%]** | **Purity [%]** | **Purity [%]** |
| R1-R7 | 0,00 | 0 | 0,00 | 0,00 | 0,00 | 0,00 |
| R8 | 1,53 | 0 | 3,34 | 0,00 | 0,00 | 0,00 |
| R9 | 2,95 | 0 | 3,77 | 0,00 | 0,00 | 0,00 |
| R10 | 13,53 | 0 | 3,33 | 1,17 | 0,00 | 0,00 |
| R11 | 66,39 | 1 | 5,86 | 8,00 | 0,00 | 0,00 |
| R12 | 87,83 | 13 | 4,11 | 2,96 | 0,04 | 0,01 |
| R13 | 88,01 | 20 | 5,22 | 0,49 | 0,03 | 0,01 |
| R14 | 91,11 | 47 | 4,12 | 0,00 | 0,03 | 0,02 |
| R15 | 91,68 | 16 | 4,19 | 0,00 | 0,07 | 0,06 |
| R16 | 86,48 | 1 | 4,11 | 0,00 | 0,50 | 0,25 |
| R17 | 51,37 | 0 | 3,72 | 0,00 | 13,73 | 2,13 |
| R18 | 1,09 | 0 | 0,13 | 0,00 | 10,41 | 76,69 |

| **Virtual combination of fraktions** | | | | | | |
|---|---|---|---|---|---|---|
| | **Tacrolimus** | | **Tautomer II** | **Tac I** | **Tac II** | **Tac III** |
| **fraction** | **Purity [%]** | **Yield [%]** | **Purity [%]** | **Purity [%]** | **Purity [%]** | **Purity [%]** |
| R13-R16 | 90,38 | 85 | 4,4 | 0,12 | 0,04 | 0,03 |
| targets | | > 60 | | 0,12 | 0,4 | 0,16 |

### Example 6: Purification of irinotecane by using the sorbent produced in Example 3:

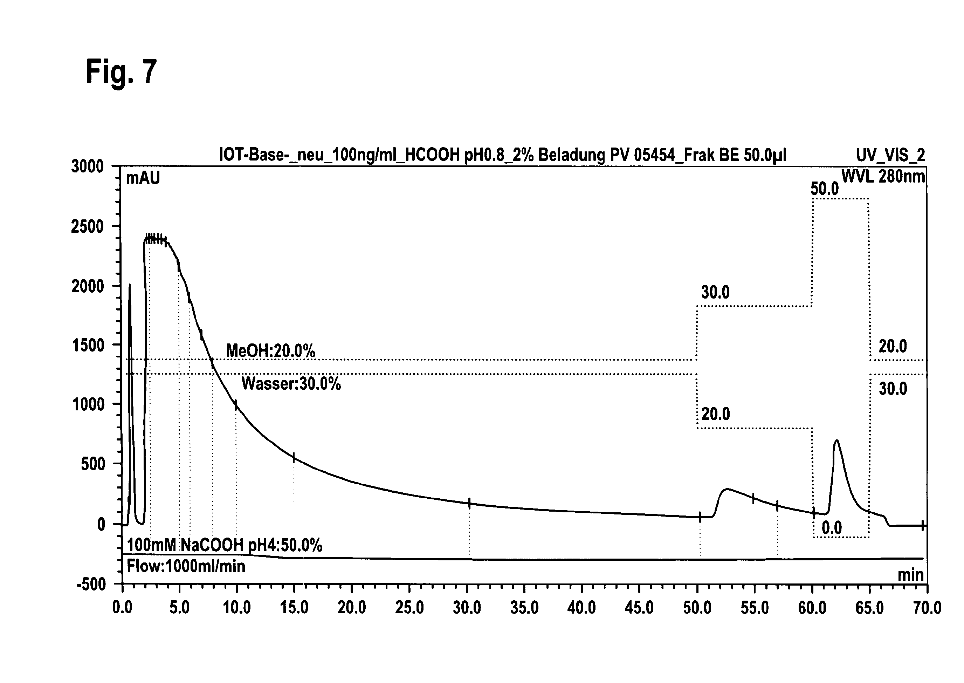

The crude mixture of irinotecane and several impurities were separated using an Dionex HPLC system consisting of a four channel low-pressure gradient pump (LPG 580, LPG 680 or LPG 3400), auto sampler (Gina 50, ASI-100 or WPS-300), six-channel column switching valves (Besta), column oven and a diode-array uv detector (UVD 170U, UVD 340S or VWD 3400). The sorbent was filled in a 33x4 mm steel column. The fractionation was performed with 20% methanol and 50 mM sodium formate buffer (pH 4) in water. Figure 7 shows the course of the fractionation. Collecting fractions 4 to 16 the product could be obtained in 98.8% purity and 89% yield.

### Example 7: Purification of partricine derivative by using the sorbent produced in Example 2:

The sorbent produced in Example 2 was packed into a 250x4 mm steel column and equilibrated with 50 mM ascorbic acid and 500 mM sodium formate (pH = 3) in 50% methanol on a Dionex 3000 system. The crude mixture containing the thiocolchicoside and several impurities were injected with a sample load of 1.0% (w/w).

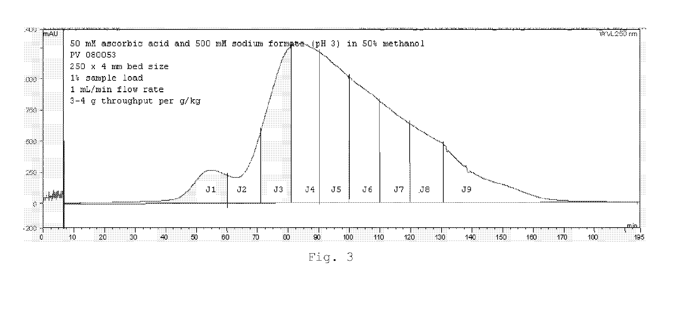

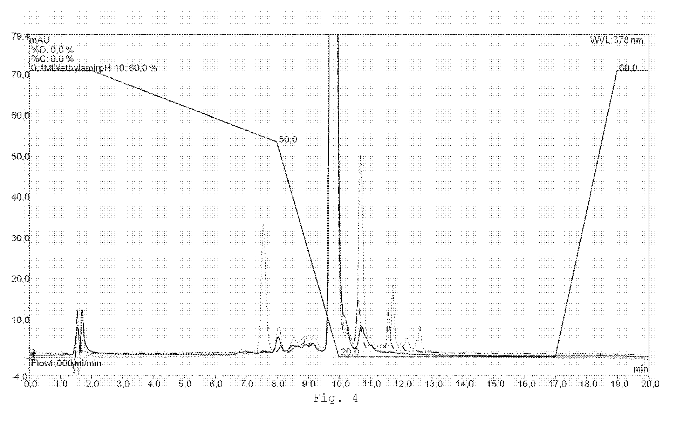

The chromatogram in Figure 3 shows the preparative run and the fractions that were collected. As be seen from Table 2 below collecting the fractions J4 to J7 gave the product in 63% yield with a purity of 96% and the two main impurities reduced below the targeted threshold. The analytical chromatogram in Figure 4 shows the reduction of the impurities from the crude mixture (short dashed line) to the purified product (continuous line). The working standard is shown as dashed and dotted line.

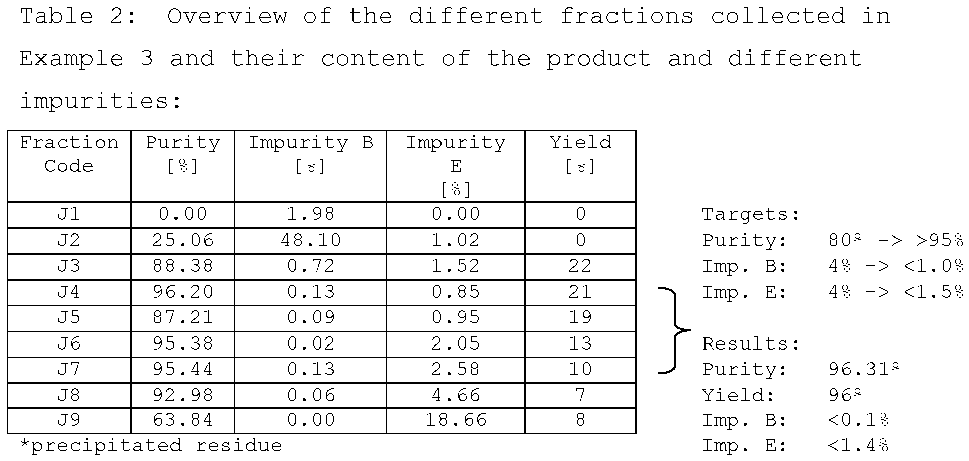

### Example 8: Purification of voglibose by using the sorbent produced in Example 4:

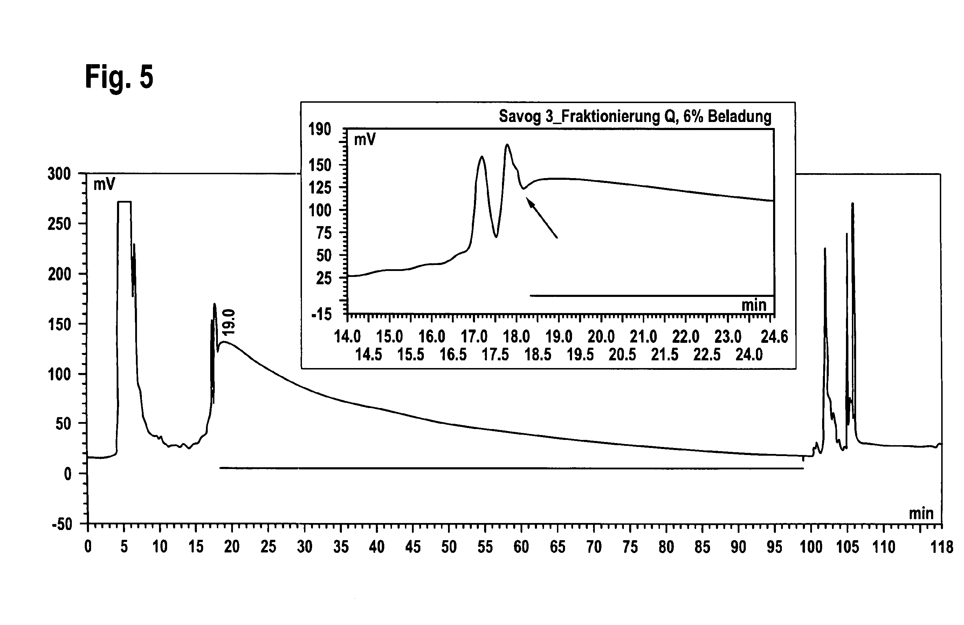

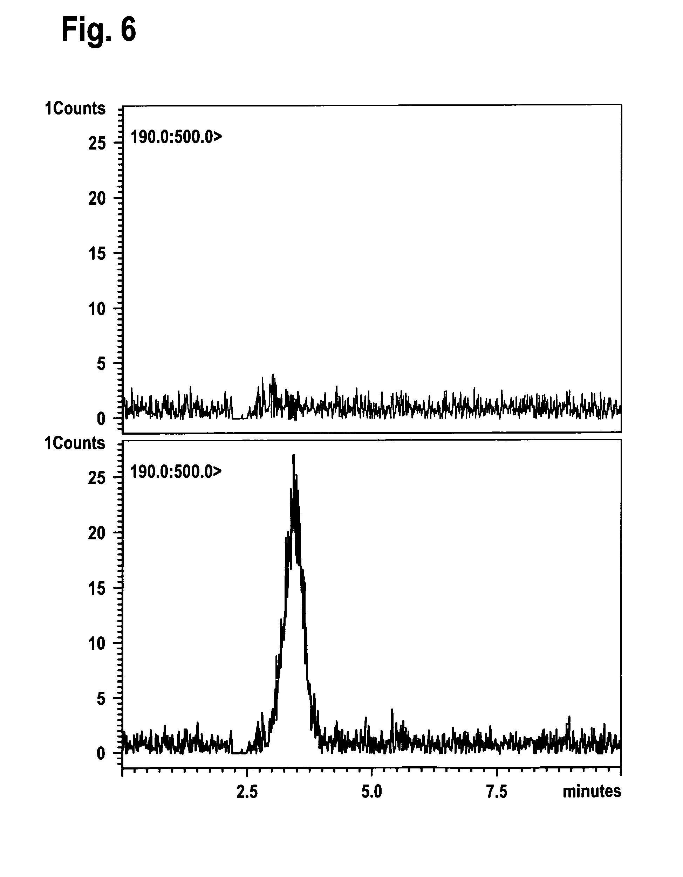

The crude mixture of voglibose and several impurities were separated using an Dionex HPLC system consisting of a four channel low-pressure gradient pump (LPG 580, LPG 680 or LPG 3400), auto sampler (Gina 50, ASI-100 or WPS-300), six-channel column switching valves (Besta), column oven and a diode-array uv detector (UVD 170U, UVD 340S or VWD 3400). The sorbent produced in Example 4 was filled in a 250x4 mm steel column. The mobile phase consisted solely of pure water. As indicated in Figure 5 the product fraction was taken after the two main impurities eluated around 17 to 19 minutes up to 99 minutes until the product peak reached baseline. The product fraction and the crude mixture were analyzed using LC-MS as shown in Figure 6a (product fraction with no impurities) and 6b (impurities). According to LC-MS the critical impurities were well depleted below the 0.047% of the standard mixture.

## Claims

1. Sorbent comprising a solid support material, the surface of which comprises a residue of the following general formula (I): 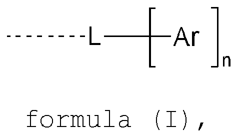 wherein the residue is attached via a covalent single bond represented by the dotted line in formula (I) to a functional group on the surface of either the bulk solid support material itself or of a polymer film on the surface of the solid support material; and
wherein the symbols and indices have the following meanings:
L is an n-valent linear aliphatic hydrocarbon group having 1 to 20 carbon atoms or branched or cyclic aliphatic hydrocarbon group having 3 to 20 carbon atoms, wherein one or more CH₂-moieties in said groups may be substituted by a CO, NH, O or S, one or more CH-moieties in said groups may be substituted by N, said groups may comprise one or more double bonds between two carbon atoms, and one or more hydrogen atoms may be substituted by D, F, Cl or OH;
Ar represents independently at each occurrence a monovalent mono- or polycyclic aromatic ring system having 6 to 28 aromatic ring atoms, wherein one or more hydrogen atoms may be substituted by D, F, Cl, OH, C₁₋₆-Alkyl, C₁₋₆-Alkoxy, NH₂, -NO₂, -B (OH)₂, -CN or -NC; and
n is an index representing the number of Ar-moieties bound to L and is 1, 2 or 3.

2. Sorbent according to claim 1, wherein L is selected from the group consisting of
- (C₁₋₁₀-alkylene) -,
- (C₁₋₆-alkylene) -NH-,
- C (O) -,
- C (O) -NH-,
- C (O) -CH (OH)-
- C (O) -NH-NH-C (O) O-,
- C (O) -(C₁₋₁₂-alkylene) -,
- C (O) -NH- (C₁₋₆-alkylene)-,
- C (O) -(C₁₋₁₂-alkylene) -C (O) -
- C (O) - (C₁₋₁₂-alkylene) -NH-C (O) O-
- C (O) - (C₁₋₆-alkylene) -C (O) -NH-,
- C (O) - (C₁₋₆-alkylene) -C (O) -NH- (C₁₋₆-alkylene)-,
- C (O) -O- (C₁₋₆-alkylene)-,
- C (O) - (C₁₋₆-alkylene) -Y-, wherein Y is NH, O or S,
- C (O) - (C₁₋₃-alkylene) -O- (C₁₋₃-alkylene) -C(O) -NH-,
- C (O) - (C₁₋₃-alkylene) -O- (C₁₋₃-alkylene) -C (O) -NH- (C₁₋₆-alkylene)-,
- C (O) - (C₁₋₆-alkylene) -C (O) -NH- (C₁₋₆-alkylene) -NH-C (O) -NH-,
- CH₂-CH (OH) -CH₂- (OCH₂CH₂)ₘ-O-,
wherein m is 1, 2, 3, 4, 5 or 6
- (C₁₋₆-alkylene) -Y- (C₁₋₆-alkylene)-,
wherein Y is S, O, NH or -S (O₂)-
- C (O) - (CH (CH₂CH(CH₃) ₂)) -NH-C (O)-,
- C (O) - NH- (C₁₋₆-alkylene) -NH-C (O)-,
- C (O) - (C₁₋₆-alkylene) -NH-C (O) - (CH (CH₂CH(CH₃) ₂))-NH-C (O)-, 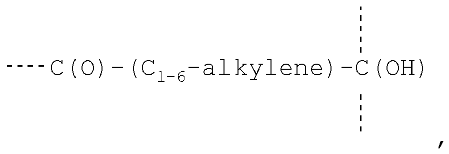 and 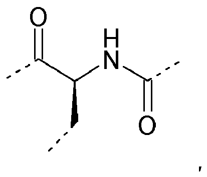 wherein the dotted lines represent the bonds to the functional group on the surface of either the bulk solid support material itself or of a polymer film on the surface of the solid support material and Ar.

3. Sorbent according to claim 1 or 2, wherein Ar is a monovalent aromatic ring system having 6 to 14 aromatic ring atoms.

4. Sorbent according to any one of claims 1 to 3, wherein Ar is substituted with one or more F or CN.

5. Sorbent according to claim 4, wherein Ar is a perfluorated aromatic ring system.

6. Sorbent according to claim 4, wherein Ar is substituted with one -CN.

7. Sorbent according to any one of claims 1 to 6, wherein n is 1.

8. Sorbent according to any one of claims 1 to 7, wherein the sorbent comprises a further residue comprising a group Pₛ, which is either a anionic or deprotonizable group, wherein the further resdidue is attached via a covalent single bond to a functional group on the surface of either the bulk solid support material itself or the polymer film on the surface of the solid support material.

9. Sorbent according to claim 8, wherein the deprotonizable group Pₛ is -SO₃H, -COOH or -PO₃H₂.

10. Sorbent according to any one of claims 1 to 9, wherein the surface of the solid support material is covered with a film of a polymer which comprises or consists of individual chains which are covalently crosslinked with each other, but which are not covalently bound to the surface of the solid support material.

11. Sorbent according to claim 10, wherein the polymer is a polyamine, a polyvinylamine, or a copolymer or a polymer blend comprising polyamine.

12. Use of a sorbent according to any one of claims 1 to 11 for the purification of organic molecules.

13. Use according to claim 12, wherein the organic molecules are pharmaceutically active compounds.

14. Use according to claim 12 or 13, wherein the organic molecules have a molecular weight in the range of from 500 to 200000 g/mol.

15. Use according to any one of claims 12 to 14, wherein the organic molecules are selected from the group consisting of partricine, tacrolimus, irinotecane, voglibose, their derivatives, and endotoxines.
